# EUROPEAN PATENT APPLICATION

(11) **EP 4 197 448 A1**
(43) Date of publication of application: **21.06.2023**
(21) Application number: 21214383.8
(22) Date of filing: 14.12.2021
(51) Int. Cl.: A61B 6/04, A61N 5/10

(54) **MEDICAL SYSTEM**

(71) Applicant: Koninklijke Philips N.V., 5656 AG Eindhoven (NL)
(72) Inventor: CHAUDHURY, Sudipta, Eindhoven (NL); PAUL, Soubhik, Eindhoven (NL); CHAKRABARTI, Biswaroop, Eindhoven (NL); VON BERG, Jens, Eindhoven (NL); WIEBERNEIT, Nataly, Eindhoven (NL); YOUNG, Stewart, Matthew, Eindhoven (NL); BRUECK, Heiner, Matthias, Eindhoven (NL)
(74) Representative: Philips Intellectual Property & Standards

(57) **Abstract**

The present disclosure provides a medical system comprising a platform for mounting a subject, the platform configured to allow for automatic adjustment of a subject position with 6 degrees of freedom and a processing system configured to determine, based on image data, whether there is a position error in the subject position and, in response to determining that there is a position error, performing a position correction procedure. The position correction procedure comprises the steps of determining a position error vector that represents the position error, transforming the position error vector to a position correction vector, and controlling the platform to automatically move in such a manner as to adjust the subject position in accordance with the position correction vector.

## Description

### FIELD OF THE INVENTION

The present disclosure provides a medical system comprising a platform for mounting a subject and a processing system, and a position correction procedure using the medical system.

### BACKGROUND OF THE INVENTION

In medical systems, for example medical systems that may be used for imaging procedures, e.g., X-ray imaging or the like, proper positioning of a subject on which the system is used, may be of critical importance, for example for proper image reconstruction and/or accurate identification of features in the images. As an example, X-ray is one of the most established first line imaging modalities that is used to investigate patients. For example chest X-ray is often used to detect abnormalities in the thorax region, especially in lungs.

Suitable patient position aspects, in particular with regard to the patient rotation, are very important requirements regarding image quality, particularly in radiograph interpretation.

In present systems and methods, errors pertaining to patient position, including rotational and translational position errors, can be detected before (e.g., by using a camera) or after acquiring a first radiograph, e.g. by analyzing various image features, and an operator can be prompted to correct the same, in some cases with patient participation.

Once the patient position error is detected, the next step is to correct the patient position and then perform the imaging. These steps may require iterative corrections, especially for elderly and frail patients that need to be moved with care, and can lead to increased scan time, additional, otherwise-unnecessary radiation exposure, and patient inconvenience. In case of some patients, e.g. ICU patients, it may be difficult to perform a proper correction with the known methods, for example due to the patient's critical and/or immobile condition.

Thus, known solutions are inefficient and lack precision and may also entail various other disadvantages outlined above.

### SUMMARY OF THE INVENTION

Accordingly, it is an object solved by the present invention to provide a medical system having improved positioning capabilities, particularly improved efficiency and/or accuracy of positioning a subject.

The invention provides a medical system, method for using a medical system, computer program product, and computer readable medium according to the independent claims. Preferred embodiments are laid down in the dependent claims.

The present disclosure provides a medical system comprising a platform for mounting a subject, the platform configured to allow for automatic adjustment of a subject position with 6 degrees of freedom, and a processing system configured to determine, based on image data, whether there is a position error in the subject position and, in response to determining that there is a position error, performing a position correction procedure. The position correction procedure comprises the steps of determining a position error vector that represents the position error, transforming the position error vector to a position correction vector, and controlling the platform to automatically move in such a manner as to adjust the subject position in accordance with the position correction vector.

The system allows for removing even small errors, including small rotation errors, that known systems may not be able to detect and/or correct. Moreover, the system does not require manual effort or patient cooperation or correcting the position iteratively.

The medical system may be employed, for example, for X-ray imaging, particularly including chest X-rays and/or narrow angle tomo-synthesis image acquisition.

The subject may, for example, be a patient, for example a patient to be subjected to an imaging procedure, e.g., X-ray imaging, for example a chest X-ray or a narrow angle tomo-synthesis.

The medical system may be employed with a patient bed and/or a patient chair, e.g., a dental chair, and/or an operation theater table that are mounted on the platform, with a subject, e.g., a patient, being mounted on the patient bed, the patient chair, or the operation theater table, respectively. The medical system may also be employed with standing-platforms for a patient to stand on, mounted on the platform. The subject, e.g., patient, may also be mounted directly on the platform itself, for example, stand on top of the platform.

The platform may be a Stewart platform, which is a multiple degrees of freedom pedestal used for simulating multi-axis movements at a fixed position.

In the present disclosure, the term position is used collectively to refer to an orientation or pose and a location. Accordingly, a position error may comprise an error in orientation, also referred to as a rotational error or angular error or tilt, and a position error may, alternatively or in addition, comprise a translational error or shift.

A position error may be described by one or more position error vectors. For example, a position can be expressed as three orthogonal vectors having a common origin. A position error may be determined on the basis of a comparison with corresponding reference vectors, e.g., three orthogonal vectors having a common origin. A translational error may be described, for example, by a position error vector pointing from the reference vector origin to the origin of the position vectors. A rotational error may be a rotation of the position vectors relative to the reference vectors and may also be expressed by means of one or more position error vectors. In the present disclosure reference vectors may, in particular, be target vectors that express the target position for the subject.

Thus, depending on the type of position error, one or more position error vectors may be obtained. For each of the position error vectors, a transformation is performed to obtain a corresponding position correction vector.

In the simplest example, transforming a position error vector to obtain a position correction vector may be to negate the position error vector. However, depending on an input expected for controlling the platform, other types of transformations of the position error vector may be applied.

Determining whether there is a position error in the subject position may comprise defining at least three landmark points (P₁, P₂, P₃) on the subject and three corresponding subject position vectors (V₁ₚ, V₂ₚ, V₃ₚ) and determining that at least one of the subject position vectors does not coincide with a corresponding reference vector, for example a reference vector on a detector that provided the image data. In particular, the determining that at least one of the subject position vectors does not coincide with a corresponding reference vector may comprise determining whether there is a deviation in angle and/or an origin shift between the subject position vectors and the corresponding reference vectors.

As an example for the corresponding subject position vectors, a first subject position vector V₁ₚ may extend from an origin Oₚ along a line connecting landmark points P₂ and P₃, a second subject position vector V₂ₚ may extend in a perpendicular orientation with respect to V₁ₚ from the origin to point P₁. A third subject position vector V₃ may extend from the origin perpendicularly to V₁ₚ and V₂ₚ. The subject position vectors are also referred to, herein, as patient vectors.

The reference vectors, as mentioned above, may represent the target position of the subject. For example, it may be a calibrated position, i.e., location and orientation, of the sensor with respect to a source (without any subject present). Accordingly, the reference vectors may be seen as may be predetermined target vectors V₁ₜ, V₂ₜ, V₃ₜ.

In particular, determining the position error vector comprises determining, for each of the subject position vectors, an angular difference between the subject position vector and the corresponding reference vector and/or an origin shift between the subject position vectors and the corresponding reference vectors.

In the present disclosure, the at least one position correction vector may represent a composite of an angular change and an origin shift or may comprise one or more first position correction vectors representing an angular change and a second position correction vector representing an origin shift.

In the present disclosure, determining whether there is a position error in the subject position may comprise defining at least three landmark points (P₁, P₂, P₃) on the subject, for example landmark points described above, and three corresponding subject position vectors (V₁ₚ, V₂ₚ, V₃ₚ), for example the subject position vectors described above. The position error may comprise an origin shift, wherein an origin shift is represented by a vector (Oₜ-Oₚ), wherein Oₜ is a target origin, which is a pre-defined 3D point representing the origin of the reference vectors, and wherein Oₚ is a/the subject position origin which is a 3D point representing the origin of the subject position vectors (V₁ₚ, V₂ₚ, V₃ₚ). The negative magnitude of the vector (OᵣOₚ) and its direction may be used as input for correction, in particular as input for a controller controlling operation of the platform.

In particular, performing a position correction may be performed by means of a feedback loop to bring and/or keep the magnitude of the vector (Oₜ - Oₚ) = 0 or within a predetermined tolerance.

In the present disclosure, determining whether there is a position error in the subject position may comprise defining at least three landmark points (P₁, P₂, P₃) on the subject and three corresponding subject position vectors (V₁ₚ, V₂ₚ, V₃ₚ). The position error may comprise a deviation in angle. The reference vectors may be predetermined target vectors V₁ₜ, V₂ₜ, V₃ₜ representing a target position (e.g., an ideal position) for the subject. For example, the medical system may comprise a radiation source and/or a detector and the target vectors may represent a target position in relation with the radiation source of the medical system and/or in relation with the detector of the medical system. Correction of the subject position may comprise a subject rotation, described by the Quaternions (V₁ₜ - V₁ₚ) = Q₁, (V₂ₜ - V₂ₚ) = Q₂, (V₃ₜ - V₃ₚ) = Q₃. Each of the Quaternions may be negated and used as input for correction, particularly as input for a controller controlling operation of the platform.

In particular, position correction may be performed by means of a feedback loop to bring and/or keep the magnitude of each Quaternion component X, Y, Z and W to be 0 or within a predetermined tolerance.

In this context, subtracting subject position vectors and reference vectors of each axis generates a Quaternion. Each Quaternion is represented by X, Y, Z, W components. Where X, Y and Z are angular displacements and W is the ratio of difference in length of the vectors.

The medical system of the present disclosure may be configured to, continuously or at predetermined times and/or at predetermined time intervals monitor a subject position, determine, based on image data, whether there is an error in the subject position, and, in response to determining that there is an error, performing the position correction procedure. In particular, the medical system is configured to perform the position correction procedure using a feedback loop.

The medical system of the present disclosure may comprise a subject support, in particular a bed and/or a chair and/or a chamber, mounted on and attached to the platform in a position that is fixed relative to the platform such that an adjustment of the position of the platform directly translates to an adjustment of a position of the subject support. This allows for various different applications and use cases in different settings.

The medical system of the present disclosure may comprise a subject stabilization structure that is configured to stabilize a subject mounted on the platform. In particular, the subject stabilization structure may be configured to surround the subject, e.g., during adjusting the subject position by means of the platform. This allows for improving positioning and, accordingly, imaging accuracy and may protect the subject during position correction. The subject stabilization structure may comprise multiple inflatable chambers, for example chambers configured to be filled with a fluid to expand the walls of the chambers. This allows for high flexibility in terms of the application or use case and the subject to be stabilized.

The medical system may comprise the subject stabilization structure and a subject support in the form of a chamber for accommodating the subject, mounted on and attached to the platform in a position that is fixed relative to the platform such that an adjustment of the position of the platform directly translates to an adjustment of a position of the subject support. The subject stabilization structure may be configured to be arranged between a subject and one or more chamber walls of the chamber and to exert pressure on one or more chamber walls and the subject so as to stabilize the subject, in particular by inflating the inflatable chambers. This allows for high flexibility in terms of the application or use case and the subject to be stabilized. At the same time, it can be avoided that excessive force is exerted on a subject.

In particular, the chamber and/or the subject stabilization structure may be at least partially transparent to X-ray radiation. Thus, it does not deteriorate image quality in an X-ray imaging use case.

The subject stabilization structure of the present disclosure may be configured to control initial inflation of the inflatable chambers so as to align with the subject contour, particularly, inflating all chambers with the same initial pressure. Alternatively or in addition, the subject stabilization structure of the present disclosure may be configured to control adjustment of the inflation of the inflatable chambers after initial inflation. In particular, the subject stabilization structure may be configured to control adjustment that compensates forces resulting from acceleration of the platform and/or forces resulting from gravity when tilting the subject with respect to a vertical direction. Alternatively or in addition, the subject stabilization structure of the present disclosure may be configured to control adjustment of the inflation on the basis of a feedback loop directed at the pressure in the chambers. For example, the subject stabilization structure may control the adjustment by means of a/the controller controlling operation of the platform.

The medical system of the present disclosure may comprise a CT imaging system and the subject is a subject to be imaged by means of the CT imaging system while mounted on the platform, in particular a patient.

The image data of the present disclosure may be CT image data and/or optical image data, e.g., obtained by a camera and/or a laser scanning system, e.g. LiDAR.

In the present disclosure, determining an error in the subject position from the image data may comprise analyzing image features in the image data.

In the present disclosure, adjusting the subject position may comprise correcting rotation errors.

The present disclosure also provides a method for using a medical system, in particular a medical system according to the present disclosure. The method comprises mounting a subject on a platform for mounting a subject, the platform configured to allow for automatic adjustment of a subject position with 6 degrees of freedom, determining, based on image data, whether there is a position error in the subject position and, in response to determining that there is a position error, performing a position correction procedure. The position correction procedure comprises the steps of determining a position error vector that represents the position error, transforming the position error vector to a position correction vector, and controlling the platform to automatically move in such a manner as to adjust the subject position in accordance with the position correction vector.

The method may further comprise continuously or at predetermined times and/or at predetermined time intervals monitoring a subject position, determining, based on image data, whether there is an error in the subject position, and, in response to determining that there is an error, performing the position correction procedure.

Alternatively or in addition, the method may comprise performing the position correction procedure using a feedback loop.

Alternatively or in addition, the method may comprise stabilizing a subject mounted on the platform, in particular during adjusting the subject position by means of the platform, using a subject stabilization structure, which comprises multiple inflatable chambers, for example chambers configured to be filled with a fluid to expand the walls of the chambers, the stabilizing comprising initially inflating the inflatable chambers so as to align with the subject contour, particularly, inflating all chambers with the same initial pressure, and/or controlling adjustment of the inflation of the inflatable chambers after initial inflation, in particular, adjustment compensating forces resulting from acceleration of the platform and/or forces resulting from gravity when tilting the subject with respect to a vertical direction.

Alternatively or in addition, the method may comprise performing narrow angle tomo-synthesis image acquisition on a subject mounted on the platform.

Alternatively or in addition, the method may comprise performing CT imaging on a subject, in particular on an immovable subject mounted on the platform, e.g., an ICU patient, and/or on a patient with a neurological disorder and/or on a patient with a cognitive disability.

Alternatively or in addition, the method may comprise adjusting operation theatre table position and/or patient bed and/or dental chair position so as to bring a region of interest of the subject mounted on the operation theatre table and/or the patient bed and/or dental chair into a predetermined position.

Alternatively or in addition, the method may comprise performing X-ray irradiation, particularly by means of a mobile X-ray unit, and account for ground irregularities and/or swaying and/or shaking and/or vibrating, e.g. due to a vehicle's suspension.

The invention also provides a computer program product comprising instructions which, when the program is executed by a computer, cause the computer to carry out and/or control one or more, in particular all of the steps of the method of the present disclosure, except steps specified as being carried out manually and/or by an operator or user.

The invention also provides a computer readable medium comprising instructions which, when executed by a computer, cause the computer to carry out and/or control one or more, in particular all of the steps of the method of the present disclosure, except steps specified as being carried out manually and/or by an operator or user.

The features and advantages outlined in the context of the medical system similarly apply to the method for using a medical system, the computer program product, and the computer readable medium of the present disclosure.

Further features, examples, and advantages will become apparent from the detailed description making reference to the accompanying drawings.

### BRIEF DESCRIPTION OF THE DRAWINGS

Fig. 1 shows a schematic and not to scale illustration of a medical system according to the present disclosure;
Fig. 2 shows a schematic and not to scale illustration of a medical system according to the present disclosure with a subject mounted on the platform;
Figs. 3a to 3c show schematic and not to scale illustrations of a medical system according to the present disclosure with a subject mounted on the platform
Fig. 4 shows a flow diagram of a method for adjusting patient position according to the present disclosure;
Fig. 5 shows a patient with patient vectors;
Fig. 6 shows patient vectors being misaligned with respect to target vectors;
Figs. 7a to 7d show an exemplary platform for 6 degree of freedom adjustment; and
Fig. 8 shows a setup of a medical system according to the present disclosure for performing narrow angle tomo-synthesis image acquisition.

### DETAILED DESCRIPTION OF EMBODIMENTS

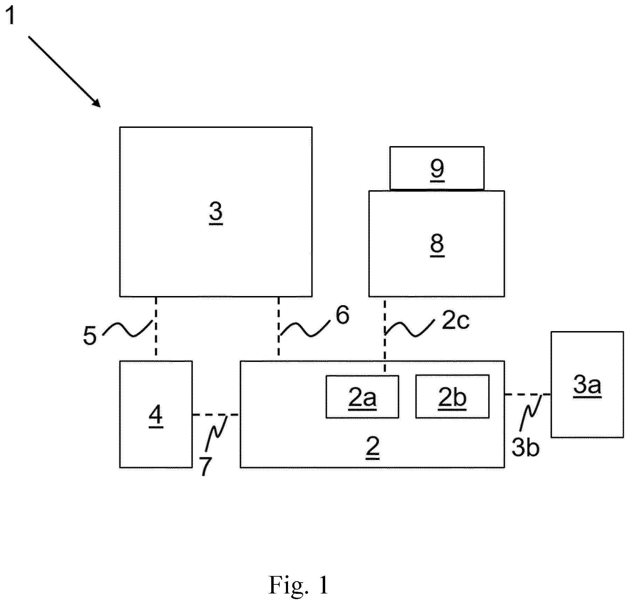

Fig. 1 shows a schematic and not to scale illustration of a medical system 1 according to the present disclosure.

The medical system comprises a processing system 2, in particular, a processing system comprising a controller 2a and other computing components 2b. The controller may be formed integrally in a device together with other computing components 2b or it may be formed in a device separate from the other computing components. The controller may control the platform by sending control signals via a data connection 2c, which may be wired or wireless. For example, a desktop computer and/or laptop and/or server may comprise at least some of the other computing components and the controller may be physically separated, for example, physically attached to or incorporated into other components of the medical system, e.g., the platform.

The medical system as shown in Fig. 1, as an example, comprises a CT imaging system 3. However other type of imaging systems are conceivable instead of or in addition to the CT imaging system.

The medical system shown in Fig. 1 comprises an optional display device 4, which may be configured to display images from the CT imaging system and/or processed images, for example.

Optional data connections 5, 6, and 7 connecting the display device, processing system, and CT imaging system are shown in Fig. 1. They may be wired and/or wireless data connections.

A platform 8 is also shown schematically in Fig. 1. The controller 2 may be configured to control the platform. In an example, where the controller is physically attached to or incorporated into other components of the medical system, it may, for example, be physically attached to or incorporated into the platform. The platform is configured to allow for automatic adjustment of a subject position with 6 degrees of freedom.

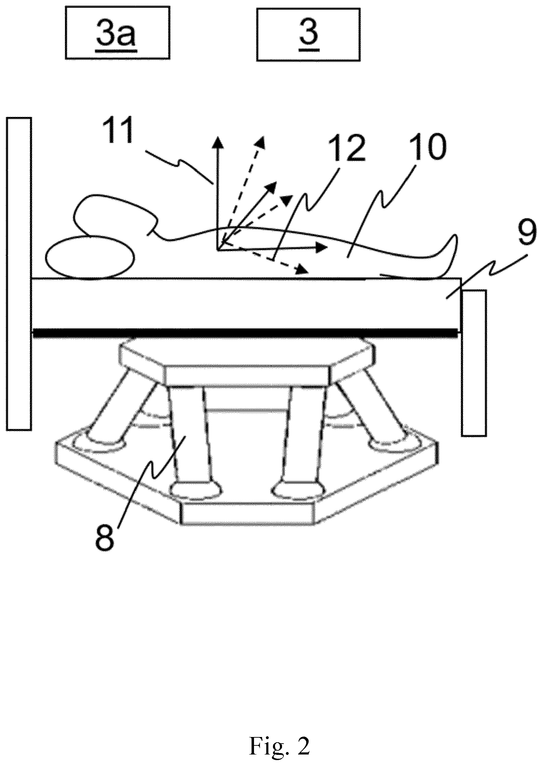

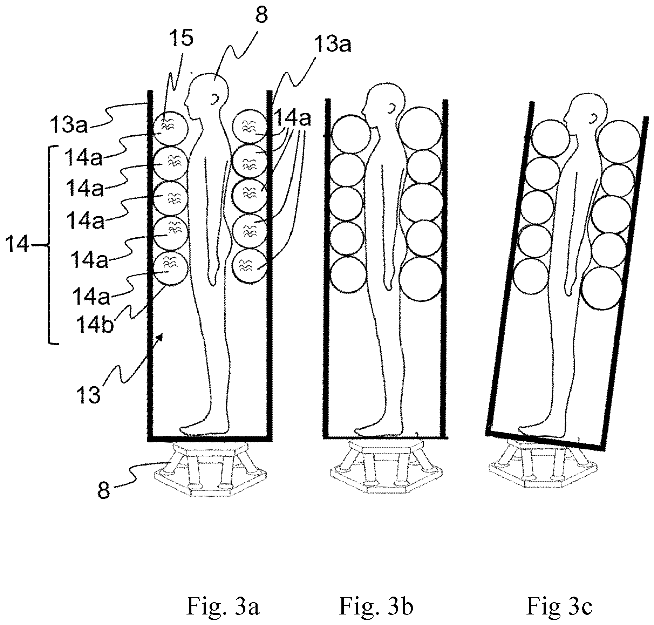

In operation, a subject (not shown in Fig. 1, reference is made to Figs. 2 or 3) may be mounted on the platform directly or indirectly via a subject support, e.g., a bed or chair or chamber.

The processing system is configured to determine, based on image data, whether there is a position error in the subject position and, in response to determining that there is a position error, perform a position correction procedure.

The image data may be obtained by the CT imaging system 3 or an additional imaging system 3a, e.g., a camera or optical scanner, optionally connected to the processing system via a data connection 3b, which may be wired or wireless.

The position correction procedure comprises the steps of determining a position error vector that represents the position error, transforming the position error vector to a position correction vector, and controlling, for example by means of the controller, the platform to automatically move in such a manner as to adjust the subject position in accordance with the position correction vector, as will be explained in the context of Fig. 2 in more detail. It is noted that the platform may, for example, comprise a drive system (not separately shown in the Figures) for moving at least some of its components based on the control signals of the controller.

Fig. 2 shows a schematic and not to scale illustration of a medical system 1 according to the present disclosure with a subject mounted on the platform. The overall configuration may, for example, correspond to that of Fig. 1. Accordingly, the above-described features of the medical system will not be explicitly repeated, and reference is made to the description of Fig. 1.

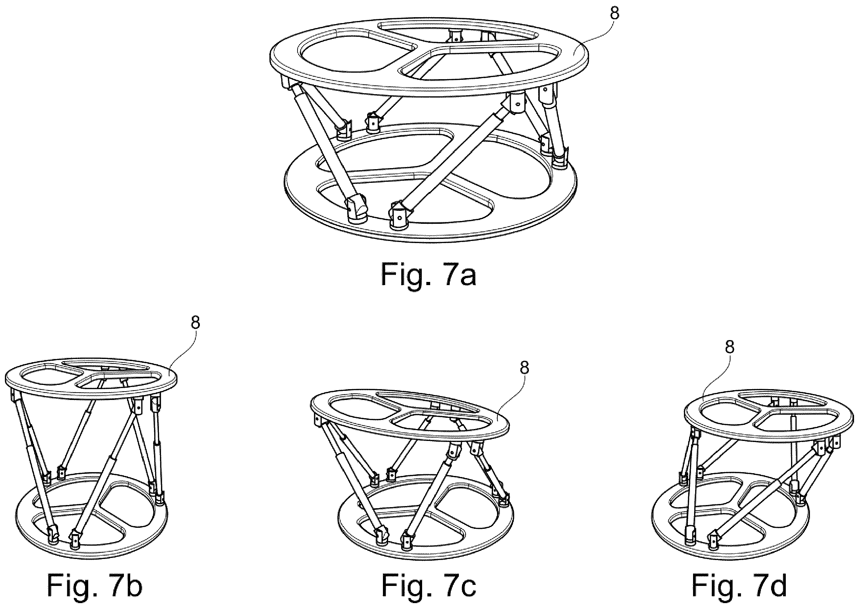

The platform may be a Stewart platform, for example. The platform can be adjusted with six degrees of freedom. For example, a platform as shown in Figs. 7a to 7d may be used. In principle, a subject could be mounted directly onto the platform. However, in the example shown in Fig. 2, the subject 10 is mounted on the platform 8 via a subject support 9. In other words, the subject is mounted on the patient support and the patient support is mounted on the platform. In the present example, the subject support is configured as a patient bed, but it may also be configured as a chair or chamber, for example.

In Fig. 2, subject position vectors 11 representing the subject's position, and target vectors 12 representing the target position for the subject are shown. For each of the three subject vectors, the position error vector can be determined with respect to a corresponding one of the three target vectors. Examples of subject position vectors 11 are, for example, vectors V1, V2, V3, V₁ₚ, V₂ₚ, and V₃ₚ described in the present disclosure. Examples of target vectors 12 are, for example V₁ₜ, V₂ₜ, V₃ₜ described in the present disclosure. They may also be referred to as reference vectors.

Figs. 3a to 3c show a schematic and not to scale illustration of another example of a medical system according to the present disclosure with a subject 10 mounted on the platform. The medical system is similar to that of Fig. 2 and reference is made to the description thereof.

Deviating from Fig. 2, the subject support comprises a chamber 13 for accommodating the subject. In particular, the chamber is configured and arranged so as to accommodate the subject in an upright, e.g., standing position. The chamber is shown as having walls, including side wall(s) 13a. The side wall(s) may entirely surround the patient. Alternatively, the side wall(s) may at least be arranged on opposing sides of the patient, in particular at two opposing sides and a third side of the patient. For example, the chamber may have a circular sidewall, or several sidewalls arranged such that the chamber has a polygonal, e.g., rectangular, outline. They need not extend completely around the subject, as explained above.

A subject stabilization structure 14 is arranged between the subject and the sidewall(s) of the chamber. In operation, the subject stabilization structure may exert a force on the sidewalls and the patient, so as to stabilize the patient position relative to the sidewalls. For example, the subject stabilization structure may comprise a plurality of inflatable chambers 14a having elastic walls 14b. The chambers 14a may be filled with fluid 15, for example a gas or a liquid, in operation. As an example, the subject stabilization structure may be configured as a chamber-array. In particular, the chamber-array may comprise multiple chambers attached to each other in a two-dimensional array. For example, the chamber-array may be formed by several adjacent rows of chambers attached to each other. The subject stabilization structure may be configured to wrapped at least partially around a patient, for example, it may be blanket-shaped or cape-shaped.

In Fig. 3a, the inflatable chambers are only slightly inflated, not all of them exerting pressure on the subject and the wall of the chamber in which the subject is arranged.

In Fig. 3b, the inflatable chambers are each inflated so as to exert pressure on the subject and the chamber. Thus, the subject stabilization structure adjusts or conforms to the contour of the subject, for example a patient.

In Fig. 3c, the platform has tilted, thereby also tilting the chamber in which the subject is arranged. The inflatable chambers are controlled so as to maintain the subject in a fixed relative orientation with respect to the wall of the tilted chamber, e.g., to prevent that due to gravity the subject's relative position changes. As can be seen in Figs. 3b and 3c, by inflating the chambers to different degrees, they can be made to conform to the shape of the part of the subject with which they interact.

Although not discernible in the cross-section of Figs. 3a to 3c, the subject stabilization structure may extend partially or fully around the subject, e.g., a patient.

More detailed examples are provided below concerning possible configurations of the subject stabilization structure and operation thereof.

The subject stabilization structure may fixate a patient during pose changes via the platform. A multi-chamber stabilization structure, MCSS, surrounding the patient may provide such a stabilization or fixation. The pressure in each chamber may be measured and the volume of each chamber may be adjusted individually. The adjustments are preferably performed automatically, for example controlled by the controller that also controls the platform.

By adjusting the pressure, for example, a compensation of the changing gravity forces acting on the patient while the platform is moving may be achieved. The chamber filling might be gas or liquid.

For supine examinations an analogue stabilization structure is possible.

Advantages of a MCSS are high speed of adjustment, flexibility in terms of different patient sizes or conditions, transparency to X-ray radiation, and patient comfort.

The chamber of the MCSS may also be referred to as balloons.

Balloons may be activated when a patient is mounted on top of the platform and the MCSS is arranged properly relative to the patient and optionally held in place. For example, the MCSS may be placed around the patient and closed. Here different geometries of the MCSS can be envisioned. A full enclosing of the patient achieves best stabilization, however, partial enclosing is also possible and may be beneficial depending on the context, e.g., because it is faster or more convenient to place. Such a partial enclosing could be a single circle of chambers around the hip and or shoulder.

Next, patient contour-based alignment of the balloons is described. After the MCSS is positioned around the patient, the chambers are inflated. The material of the chamber walls is elastic so that an inflation with constant pressure to all chambers would lead the different chamber volumes that align with the patient contour. In an arrangement as shown in Fig. 3b, once inflated, all balloons/chambers exert the same horizontal force onto the patient. This force stabilizes the patient. Since the expansion volume of the balloons may be limited, special configurations for children or persons or with unusual high BMI might be provided. For such cases, scaled versions of the MCSS may be provided.

A potential feedback mechanism for the MCSS is described next. When the platform starts to position the patient, e.g., for X-ray examination, as an example two types of forces may need to be compensated by the MCSS in order to keep the patient fixed relative to the platform, i.e., acceleration and gravity. However, in some cases, acceleration forces might be neglectable if the platform moves gently and gravity changes may be neglectable in case there is no, or only little tilting involved.

If the platform is tilted the patient will need more support on the lower side as the vector of gravity is not in line with the patient standing axis anymore, for example as shown in Fig. 3c. Such a change in support by the MCSS may be achieved via a feedback loop of measuring and adjusting each chamber pressure. When the platform with the patient is tilted, the chamber pressures of the upper side of the patient decrease while the chamber pressures of the lower side of the patient increase. This is detected when measuring the chamber pressures. Subsequently the controller may increase the chamber pressures of the lower side even further, thereby pushing the patient upward into the chambers of the upper side, which will lead to an increase in the chamber pressures of the upper side. Using control theory (e.g., a PID controller) as stable state can be achieved in which the position of the patient remains stable relative to the platform.

A similar system and method can be employed when a subject is arranged on a bed and the bed is moved without tilting to avoid relative movement of the subject or part thereof relative to the platform due to acceleration forces. Moreover, the stabilization structure may also serve to suppress active movement by a patient.

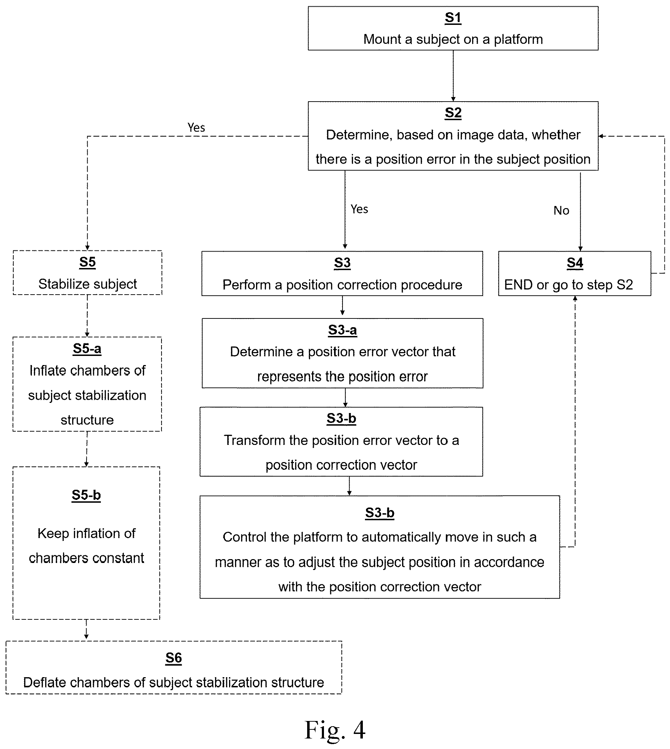

Fig. 4 shows a flow diagram of use of a medical system, for example one of the systems of the present disclosure, particularly one of the systems described above, for adjusting patient position according to the present disclosure.

In step S1, the subject is mounted on a platform, either directly or via a subject support, e.g., a bed or chair or a chamber or the like, mounted on the platform.

In step S2, it is determined, based on image data, whether there is a position error in the subject position. The method may comprise capturing the image data or accessing the image data from a storage medium.

In step S3, in response to determining that there is a position error, a position correction procedure is performed. Step S3 may comprise several steps S3-a to S3-c. In step S3-a, a position error vector that represents the position error is determined. In step S3-b the position error vector is transformed to a position correction vector. In step S3-c the platform is controlled to automatically move in such a manner as to adjust the subject position in accordance with the position correction vector.

In step S4 the method may end, for example, when a one-time correction procedure is performed. Alternatively, the method may go back to step S2 when a continued correction procedure is performed. Going back to step S2 may be performed continuously or at predetermined intervals or times. Going back to step S2 can be seen as a tracking of the subject position. Merely for the sake of completeness it is noted that step S4 may also be performed when it is determined, in step S3, that no correction is required.

Fig. 4 also shows optional step S5 of stabilizing the subject. For example, in sub-step S5-a, chambers of a subject stabilization structure are inflated to an initial inflation. In sub-step S5-b, the inflation of the chambers is kept constant, particularly while the platform moves to adjust the subject position. Thereby, the subject is stabilized during movement. In optional step S6, the chambers of the subject stabilization structure are deflated, for example, when the subject is to unmount the platform or re-positioned relative to the platform.

The above steps may be performed by any system of the present disclosure, particularly one of the systems of Figs. 1 to 3.

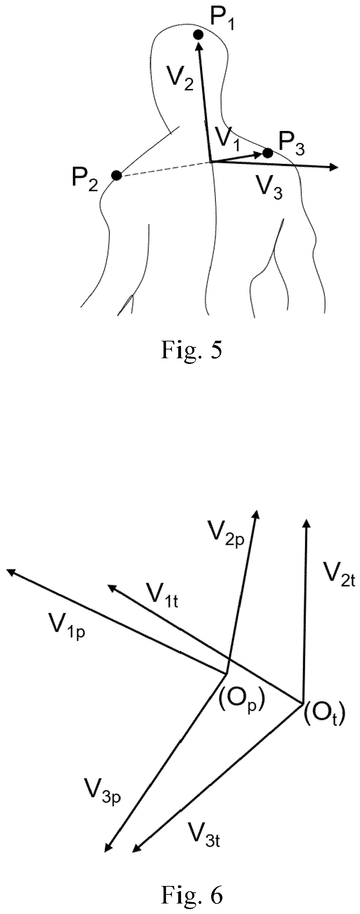

Fig. 5 shows in more detail how points and patient vectors, which are examples for subject position vectors, can be arranged on a patient. A first vector V₁ extends from an origin along a line connecting landmark points P₂ and P₃, a second vector V₂ extends in a perpendicular orientation from the origin to point P₁ and a third vector V₃ extends from the origin perpendicularly to V₁ and V₂.

Fig. 6 shows patient vectors V₁ₚ, V₂ₚ, and V₃ₚ, which are examples for subject position vectors, shifted and rotated compared to target vectors V₁ₜ, V₂ₜ, and V₃ₜ. The shift can be seen from the patient vector origin (Oₚ) not coinciding with the target vector origin (Ot).

Each vector V₁ₚ, V₂ₚ, and V₃ₚ can have either and angular movement or shift due to origin shifting. Practically the shifts may be a composite of both the movements, which can be corrected individually.

When it comes to the origin shift, the target origin (Ot), with respect to the detector is a pre-defined 3D point and a displacement with respect origin of the patient vectors (Oₚ) is the vector (Oₜ - Oₚ). As an example, for the correction, the magnitude of the vector (Oₜ - Oₚ) and its negative direction may be sent to the controller controlling the operation of the platform as input for correction. This process may be applied in a feedback loop to ensure that the magnitude of the vector (Oₜ - Oₚ) = 0 or within an accepted tolerance.

When it comes to an angular shift, target vectors V₁ₜ, V₂ₜ, V₃ₜ, which are predefined vectors with respect to best patient orientation, may be used, also referred to as reference vectors in the present disclosure. The patient rotation Quaternions (V₁ₜ - V₁ₚ) = Q₁, (V₂ₜ - V₂ₚ) = Q₂, (V₃ₜ - V₃ₚ) = Q₃, are obtained. Each Quaternions are negated and sent to the controller as input for correction. This process may also be applied in a feedback loop to ensure that the magnitude of each Quaternion components X, Y, Z and W are 0 or within an accepted tolerance.

Figs. 7a to 7d show, from left to right, an exemplary platform 8, for example a Stewart platform, in a neutral position, with a height adjustment, with a tilt/angular adjustment, and with a lateral adjustment. The different adjustments may be combined. Thus, it can be seen that 6 degrees of freedom position adjustment can be achieved.

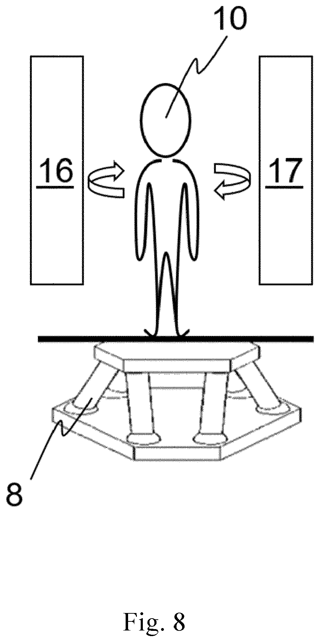

Fig. 8 shows a potential setup of a medical system according to the present disclosure for performing narrow angle tomo-synthesis image acquisition using X-ray source 16 and X-ray detector 17. The system of the present disclosure allows for achieving the tomo-synthesis partial volume at various angular intervals.

Merely for the sake of completeness, it is to be understood that the system and method of the present disclosure may also advantageously be applied for immobile patients, e.g. ICU patients, patients with neurological disorder, and/or patients with cognitive disability, and/or in the trauma department the requires carefully dealing with immobilized and non-responding patients. In said cases, it is all the more important to have an efficient and precise positioning system and method.

Similarly, the system and method of the present disclosure may also advantageously be used with operation theatre tables on which the subject is mounted. For example, it may be advantageous to adjust patient bed position to provide better/easy access to a region of interest.

Similarly, the system and method of the present disclosure may also advantageously be used with dental chairs on which the subject is mounted to adjust patient position, so that the dentist has better access to a region of interest.

Similarly, the system and method of the present disclosure may also advantageously be used for an X-ray unit deployed in a mobile van. In case the mobile van cannot be parked on a level ground and/or sways, shakes and/or vibrates, e.g. due to the vehicle's suspension, the system allows to ensure the patient platform stays correctly positioned, e.g., stays horizontal.

Similarly, the system and method of the present disclosure may also advantageously be used for body balance and posture training.

An exemplary method for using a system according to the present disclosure is provided below. The method is directed at automatically adjusting/correcting a patient position during the acquisition of X-ray images of a patient mounted on a patient bed or on patient standing platform, which in turn is mounted on a platform with 6 degrees of freedom autocorrection, for example a Stewart platform. The correction can be achieved both prior to and post-acquisition of an X-ray image, depending on the quality detection system/software in place. For example a camera/LiDAR based system may be used to detect a positional error prior to X-ray acquisition. Alternatively, image quality may be assessed post X-ray acquisition.

The first step is to detect the patient mal-positioning, e.g., shifts or rotation errors, during the X-ray acquisition. This position error detection can be prospective or retrospective with respect to image acquisition.

For a prospective position error detection, a camera/LiDAR-based system can be used to detect the (mal-)positioning of patient. A set of parameters/vectors representing the position error, including shift and rotation errors, can be derived from images obtained with the camera/LiDAR-based system. For a retrospective position error detection, i.e., post acquisition of the patient X-ray, the X-ray image is processed and analyzed to detect quality issues with respect to the position.

Once the position errors are detected, the parameters/vectors representing the error can be calculated for further processing. The position error vectors are then transformed to position correction vectors used as input for adjusting the platform.

As an example, the camera/LiDAR-based system is used to define three landmark points (P1, P2, P3) on the patient body and three corresponding vectors V1, V2, V3 (see also Fig. 5). These three vectors represent the patient position. These vectors are examples for subject position vectors V₁ₛ, V₂ₛ, V₃ₛ. If there is a positional error, it may be reflected as angular difference of the patient vectors with respect to a corresponding one of three reference or target vectors on the detector. These angular differences may be transformed to position correction vectors passed as input to the Stewart platform for further adjustment of position.

The patient position can then be corrected by adjusting the platform in up to six degrees of freedom, depending on the position correction vector, for example using a Stewart platform. For example, the adjusting is performed for the purpose of patient position correction before the X-ray exposure. Examples for various possible correctional movements of the platform for relevant positional nudging are depicted in Figs. 7a to 7d and have been described above. The adjustment of platform is driven by the position correction vectors. As the platform is adjusted based on the input position correction vectors, the patient position gets corrected accordingly and the patient position errors, including potential rotation and/or translation errors, are addressed.

After an adjustment is made, the system may continuously monitor and correct in real time, e.g., until the X-ray exposure.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

### LIST OF REFERENCE SIGNS:

| | | |
|---|---|---|
| Medical system | | 1 |
| Processing system | | 2; |
| Controller | | 2a; |
| Other computing components | | 2b; |
| Data connection | | 2c, |
| CT imaging system | | 3 |
| Imaging system | | 3a; |
| Data connection | | 3b; |
| Display device | | 4 |
| Data connections | | 5, 6, 7 |
| Platform | | 8; |
| Subject support | | 9 |
| Subject | | 10; |
| Subject vectors | | 11; |
| Target vectors | | 12; |
| Chamber for accommodating the subject | | 13; |
| Walls of the chamber for accommodating the subject | | 13a; |
| Subject stabilization structure | | 14; |
| Inflatable chambers | | 14a; |
| Walls of the chambers | | 14b; |
| Fluid | | 15; |
| X-ray source | | 16; |
| X-ray detector | | 17; |
| S1 | Mount subject on a platform; | |
| S2 | Determine, based on image data, whether there is a position error in the subject position; | |
| S3 | Perform a position correction procedure; | |
| S3-a | Determine a position error vector that represents the position error; | |
| S3-b | Transform the position error vector to a position correction vector; | |
| S3-c | Control the platform to automatically move in such a manner as to adjust the subject position in accordance with the position correction vector; | |
| S4 | END or go to step S2; | |
| S5 | Stabilize the subject; | |
| S5-a | inflate chambers of a subject stabilization structure; | |
| S5-b | keep the inflation of the chambers constant; | |
| S6 | deflate the chambers of the subject stabilization structure. | |

## Claims

1. A medical system (1) comprising
a platform (8) for mounting a subject (10), the platform (8) configured to allow for automatic adjustment of a subject position with 6 degrees of freedom; and
a processing system (2) configured to determine, based on image data, whether there is a position error in the subject position and, in response to determining that there is a position error, performing a position correction procedure comprising the steps of:
determining a position error vector that represents the position error,
transforming the position error vector to a position correction vector, and
controlling the platform (8) to automatically move in such a manner as to adjust the subject position in accordance with the position correction vector.

2. The medical system (1) according to claim 1,
wherein determining whether there is a position error in the subject position comprises defining at least three landmark points (Pi, P₂, P₃) on the subject and three corresponding subject position vectors (V₁ₚ, V₂ₚ, V₃ₚ) and determining that at least one of the subject position vectors does not coincide with a corresponding reference vector, for example a reference vector on a detector that provided the image data, in particular, whether there is a deviation in angle and/or an origin shift between the subject position vectors and the corresponding reference vectors.

3. The medical system (1) according to claim 2,
wherein determining the position error vector comprises determining, for each of the subject position vectors, an angular difference between the subject position vector and the corresponding reference vector and/or an origin shift between the subject position vectors and the corresponding reference vectors.

4. The medical system (1) according to claim 2 or 3,
wherein the at least one position correction vector represents a composite of an angular change and an origin shift or comprises one or more first position correction vectors representing an angular change and a second position correction vector representing an origin shift.

5. The medical system (1) according to any of the preceding claims,
wherein determining whether there is a position error in the subject position comprises defining at least three landmark points (P₁, P₂, P₃) on the subject and three corresponding subject position vectors (V₁ₚ, V₂ₚ, V₃ₚ),
wherein the position error comprises an origin shift,
wherein an origin shift is represented by a vector (Oₜ-Oₚ), wherein Ot is a target origin, which is a pre-defined 3D point representing the origin of the reference vectors, and wherein Oₛ is a subject position origin, which is a 3D point representing the origin of the subject position vectors (V₁ₚ, V₂ₚ, V₃ₚ), and
wherein the negative magnitude of the vector (Oₜ-Oₚ) and its direction is used as input for correction, in particular as input for a controller controlling operation of the platform,
in particular, wherein performing a position correction is performed by means of a feedback loop to bring and/or keep the magnitude of the vector (Oₜ - Oₚ) = 0 or within a predetermined tolerance.

6. The medical system (1) according to any of the preceding claims,
wherein determining whether there is a position error in the subject position comprises defining at least three landmark points (Pi, P₂, P₃) on the subject and three corresponding subject position vectors (V₁ₚ, V₂ₚ, V₃ₚ),
wherein the position error comprises a deviation in angle,
wherein the reference vectors are predetermined target vectors V₁ₜ, V₂ₜ, V₃ₜ representing a target position for a subject, for example a target position in relation with a radiation source of the medical system (1) and/or in relation with a/the detector of the medical system (1),
wherein correction of the subject position comprises a subject rotation, described by the Quaternions (V₁ₜ - V₁ₚ) = Q₁, (V₂ₜ - V₂ₚ) = Q₂, (V₃ₜ - V₃ₚ) = Q₃, and
wherein each of the Quaternions is negated and used as input for correction, particularly as input for a controller controlling operation of the platform,
in particular, wherein position correction is performed by means of a feedback loop to bring and/or keep the magnitude of each Quaternion component X, Y, Z and W to be 0 or within a predetermined tolerance.

7. The medical system (1) according to any of the preceding claims, configured to, continuously or at predetermined times and/or at predetermined time intervals:
monitor a subject position;
determine, based on image data, whether there is an error in the subject position; and,
in response to determining that there is an error, performing the position correction procedure,
in particular, wherein the medical system (1) is configured to perform the position correction procedure using a feedback loop.

8. The medical system (1) according to any of the preceding claims,
wherein the medical system (1) comprises a subject support (9), in particular a bed and/or a chair and/or a chamber, mounted on and attached to the platform (8) in a position that is fixed relative to the platform (8) such that an adjustment of the position of the platform (8) directly translates to an adjustment of a position of the subject support (9).

9. The medical system (1) according to any of the preceding claims,
comprising a subject stabilization structure (14) that is configured to stabilize a subject (10) mounted on the platform, in particular configured to surround the subject (10), in particular during adjusting the subject position by means of the platform,
wherein the subject stabilization structure (14) comprises multiple inflatable chambers (14a), for example chambers configured to be filled with a fluid (15) to expand the walls (14b) of the chambers.

10. The medical system (1) according to claim 9,
wherein the medical system (1) comprises a subject support (9) in the form of a chamber (13) for accommodating the subject (10), mounted on and attached to the platform (8) in a position that is fixed relative to the platform (8) such that an adjustment of the position of the platform (8) directly translates to an adjustment of a position of the subject support (9), and
wherein the subject stabilization structure (14) is configured to be arranged between a subject (10) and one or more chamber walls (13a) of the chamber and to exert pressure on one or more chamber walls (13a) and the subject (10) so as to stabilize the subject (10), in particular by inflating the inflatable chambers (14a),
in particular, wherein the chamber (13) and/or the subject stabilization structure (14) are at least partially transparent to X-ray radiation.

11. The medical system (1) according to any one of claims 8 to 10,
wherein the subject stabilization structure (14) is configured to:
control initial inflation of the inflatable chambers (14a) so as to align with the subject contour, particularly, inflating all chambers (14a) with the same initial pressure; and/or
control adjustment of the inflation of the inflatable chambers (14a) after initial inflation, in particular, control adjustment that compensates forces resulting from acceleration of the platform (8) and/or forces resulting from gravity when tilting the subject (10) with respect to a vertical direction,
in particular, control adjustment of the inflation on the basis of a feedback loop directed at the pressure in the chambers (14a).

12. The medical system (1) according to any of the preceding claims,
wherein the medical system (1) comprises a CT imaging system (3) and the subject (10) is a subject to be imaged by means of the CT imaging system while mounted on the platform (8), in particular a patient;
wherein the image data is CT image data and/or optical image data, e.g., obtained by a camera (3a) and/or a laser scanning system, and/or
wherein determining an error in the subject position from the image data comprises analyzing image features in the image data, and/or
wherein the adjusting the subject position comprises correcting rotation errors.

13. A method for using a medical system (1), in particular a medical system (1) according to any of the preceding claims, the method comprising mounting a subject (10) on a platform (8) for mounting a subject, the platform configured to allow for automatic adjustment of a subject position with 6 degrees of freedom, determining, based on image data, whether there is a position error in the subject position and, in response to determining that there is a position error, performing a position correction procedure comprising the steps of determining a position error vector that represents the position error, transforming the position error vector to a position correction vector, and controlling the platform (8) to automatically move in such a manner as to adjust the subject position in accordance with the position correction vector;
in particular, the method comprising:
continuously or at predetermined times and/or at predetermined time intervals monitoring a subject position, determining, based on image data, whether there is an error in the subject position, and, in response to determining that there is an error, performing the position correction procedure; and/or
performing the position correction procedure using a feedback loop; and/or
stabilizing a subject (10) mounted on the platform (8), in particular during adjusting the subject position by means of the platform (8), using a subject stabilization structure (14), which comprises multiple inflatable chambers (14a), for example chambers (14a) configured to be filled with a fluid (15) to expand the walls of the chambers (14a), the stabilizing comprising initially inflating the inflatable chambers (14a) so as to align with the subject contour, particularly, inflating all chambers (14a) with the same initial pressure, and/or
controlling adjustment of the inflation of the inflatable chambers (14a) after initial inflation, in particular, adjustment compensating forces resulting from acceleration of the platform (8) and/or forces resulting from gravity when tilting the subject (10) with respect to a vertical direction; and/or
performing narrow angle tomo-synthesis image acquisition on a subject (10) mounted on the platform (8); and/or
performing CT imaging on a subject (10), in particular on an immovable subject mounted on the platform (8), e.g., an ICU patient, and/or on a patient with a neurological disorder and/or on a patient with a cognitive disability; and/or
adjusting operation theatre table position and/or patient bed and/or dental chair position so as to bring a region of interest of the subject (10) mounted on the operation theatre table and/or the patient bed and/or dental chair into a predetermined position; and/or
performing X-ray irradiation, particularly by means of a mobile X-ray unit, and account for ground irregularities and/or swaying and/or shaking and/or vibrating, e.g. due to a vehicle's suspension, by means of the position correction procedure.

14. A computer program product comprising instructions which, when the program is executed by a computer, cause the computer to carry out and/or control one or more, in particular all of the steps of claim 13.

15. A computer readable medium comprising instructions which, when executed by a computer, cause the computer to carry out and/or control one or more, in particular all of the steps of claim 13.
